# EUROPEAN PATENT APPLICATION

(11) **EP 4 810 465 A1**
(43) Date of publication of application: **23.09.2026**
(21) Application number: 25854386.7
(22) Date of filing: 07.08.2025
(51) Int. Cl.: C07C 19/08, C07C 17/42, C07C 21/18

(54) **COMPOSITION CONTAINING 1,1,1,2-TETRAFLUOROETHANE AND METHOD FOR STORING SAME**

(30) Priority: 15.08.2024 JP 2024135676; 28.08.2024 JP 2024146590; 02.09.2024 JP 2024150887
(71) Applicant: DAIKIN INDUSTRIES, LTD., Osaka-shi, Osaka 530-0001 (JP)
(72) Inventor: ETO, Yusuke, Osaka-Shi, Osaka 530-0001 (JP); NAKAMURA, Shingo, Osaka-Shi, Osaka 530-0001 (JP); KUROKI, Yoshichika, Osaka-Shi, Osaka 530-0001 (JP); SUGIYAMA, Akinari, Osaka-Shi, Osaka 530-0001 (JP)
(74) Representative: Hoffmann Eitle
(86) International application number: PCT/JP2025/028128
(87) International publication number: WO 2026/038523

(57) **Abstract**

The present invention provides a composition comprising 1,1,1,2-tetrafluoroethane and a fluorocarbon compound having one or more double bonds, wherein an oxygen concentration, a hydrogen fluoride concentration, or a hydrogen chloride concentration is 5.0 ppm by volume or less.

## Description

### Technical Field

The present disclosure relates to a composition including 1,1,1,2-tetrafluoroethane, and a storage method thereof.

### Background Art

A fluorocarbon compound having a double bond can be included as an impurity in 1,1,1,2-tetrafluoroethane.

### Summary of Invention

### Technical Problem

It has been found that a fluorocarbon compound having a double bond included in a composition including 1,1,1,2-tetrafluoroethane may be converted into other compounds during storage to reduce the purity of 1,1,1,2-tetrafluoroethane.

An object of the present disclosure is to provide a composition in which a decrease in the concentration of 1,1,1,2-tetrafluoroethane during storage is suppressed.

### Solution to Problem

The present disclosure includes the following aspects.
[Item 1]
   A composition including 1,1,1,2-tetrafluoroethane and a fluorocarbon compound having one or more double bonds, wherein an oxygen concentration, a hydrogen fluoride concentration, or a hydrogen chloride concentration is 5.0 ppm by volume or less.
[Item 2]
   The composition according to Item 1, wherein the oxygen concentration is 5.0 ppm by volume or less.
[Item 3]
   The composition according to Item 1, wherein the hydrogen fluoride concentration is 5.0 ppm by volume or less.
[Item 4]
   The composition according to Item 1, wherein the hydrogen chloride concentration is 5.0 ppm by volume or less.
[Item 5]
   The composition according to any one of Items 1 to 4, wherein the fluorocarbon compound having one or more double bonds has one double bond.
[Item 6]
   The composition according to any one of Items 1 to 5, wherein the fluorocarbon compound having one or more double bonds has 2 or 3 carbon atoms.
[Item 7]
   The composition according to any one of Items 1 to 6, wherein the fluorocarbon compound having one or more double bonds includes at least one compound selected from the group consisting of (E/Z)1-chloro-1,2-difluoroethene, (E/Z)1-chloro-2-fluoroethene, and 3,3,3-trifluoropropene.
[Item 8]
   The composition according to any one of Items 1 to 7, wherein the oxygen concentration is 0.1 ppm by volume or more and 5.0 ppm by volume or less.
[Item 9]
   The composition according to any one of Items 1 to 8, wherein the oxygen concentration is 0.1 ppm by volume or more and 1.0 ppm by volume or less.
[Item 10]
   The composition according to any one of Items 1 to 9, wherein a concentration of the fluorocarbon compound having one or more double bonds is 0.1 ppm by volume or more and 50 ppm by volume or less.
[Item 11]
   The composition according to any one of Items 1 to 10, wherein a concentration of the fluorocarbon compound having one or more double bonds is 0.1 ppm by volume or more and 2.0 ppm by volume or less.
[Item 12]
   The composition according to any one of Items 1 to 11, wherein a concentration of the 1,1,1,2-tetrafluoroethane is 99.9999% by volume or more.
[Item 13]
   A method for storing a composition including 1,1,1,2-tetrafluoroethane and a fluorocarbon compound having one or more double bonds, wherein an oxygen concentration, a hydrogen fluoride concentration, or a hydrogen chloride concentration is set to 5.0 ppm by volume or less.
[Item 14]
   The storage method according to Item 13, wherein the oxygen concentration, the hydrogen fluoride concentration, or the hydrogen chloride concentration is 0.1 ppm by volume or more and 5.0 ppm by volume or less.
[Item 15]
   The storage method according to Item 13 or Item 14, wherein a storage temperature is 50°C or less.
[Item 16]
   The storage method according to any one of Items 13 to 15, wherein a storage temperature is 0°C or more and 50°C or less.

### Advantageous Effect of Invention

According to the composition of the present disclosure, a reduction in the concentration of 1,1,1,2-tetrafluoroethane is suppressed.

### Description of Embodiments

The composition of the present disclosure includes 1,1,1,2-tetrafluoroethane and a fluorocarbon compound having one or more double bonds, in which the oxygen concentration, the hydrogen fluoride concentration, or the hydrogen chloride concentration is 5.0 ppm by volume or less.

In one embodiment, the oxygen concentration is 5.0 ppm by volume or less.

In another embodiment, the hydrogen fluoride concentration is 5.0 ppm by volume or less.

In another embodiment, the hydrogen chloride concentration is 5.0 ppm by volume or less.

In a preferred embodiment, any two of concentrations of the oxygen concentration, the hydrogen fluoride concentration and the hydrogen chloride concentration are each 5.0 ppm by volume or less. For example, the hydrogen fluoride concentration and the hydrogen chloride concentration are each 5.0 ppm by volume or less, the oxygen concentration and the hydrogen chloride concentration are each 5.0 ppm by volume or less, or the oxygen concentration and the hydrogen fluoride concentration is 5.0 ppm by volume or less.

In a more preferred embodiment, the oxygen concentration, the hydrogen fluoride concentration and the hydrogen chloride concentration are each 5.0 ppm by volume or less.

A fluorocarbon compound having a double bond can be included in a mixed gas including 1,1,1,2-tetrafluoroethane. Such a fluorocarbon compound can be converted into other compounds during storage to reduce the concentration of 1,1,1,2-tetrafluoroethane. The composition including 1,1,1,2-tetrafluoroethane of the present disclosure has an oxygen concentration of 5.0 ppm by volume or less, and thus a fluorocarbon compound having a double bond can be inhibited from being decomposed and/or polymerized into other compounds.

The fluorocarbon compound having a double bond is typically hydrofluoroolefin (HFO).

Examples of the hydrofluoro-olefin (HFO) include 2-chloro-1,1-difluoroethene (HFO-1122), (E/Z)1-chloro-1,2-difluoroethene (HFO-1122a), trifluoroethene (HFO-1123), (E/Z)1-chloro-2-fluoroethene (HFO-1131), 2,3,3,3-tetrafluoropropene (HFO-1234yf), 3,3,3-trifluoropropene (HFO-1243zf), trans-1,3,3,3-tetrafluoropropene (HFO-1234ze), and 1,3,3,3-tetrafluoropropene (HFO-1234zd).

In one embodiment, the fluorocarbon compound having a double bond has one double bond.

In one embodiment, the fluorocarbon compound having a double bond has 2 or 3 carbon atoms.

In a preferred embodiment, the fluorocarbon compound having a double bond includes at least one compound selected from the group consisting of (E/Z)1-chloro-1,2-difluoroethene, (E/Z)1-chloro-2-fluoroethene, and 3,3,3-trifluoropropene. The fluorocarbon compound having a double bond is preferably selected from 1-chloro-1,2-difluoroethene, (E/Z)1-chloro-2-fluoroethene, and 3,3,3-trifluoropropene.

The oxygen concentration in the composition is 5.0 ppm by volume or less, preferably 3.0 ppm by volume or less, more preferably 1.0 ppm by volume or less. An oxygen concentration of 5.0 ppm by volume or less can inhibit the fluorocarbon compound having a double bond from being converted into other compounds. The oxygen concentration is preferably 0.1 ppm by volume or more, and can be, for example, 0.2 ppm by volume or more or 0.3 ppm by volume or more. The oxygen concentration can be 0.1 to 5.0 ppm by volume, preferably 0.1 to 3.0 ppm by volume, more preferably 0.1 to 1.0 ppm by volume, for example, 0.2 to 3.0 ppm by volume, 0.3 to 3.0 ppm by volume, 0.2 to 1.0 ppm by volume, or 0.3 to 1.0 ppm by volume.

The hydrogen fluoride concentration in the composition is 5.0 ppm by volume or less, preferably 3.0 ppm by volume or less, more preferably 1.0 ppm by volume or less. A hydrogen fluoride concentration of 5.0 ppm by volume or less can inhibit the fluorocarbon compound having a double bond from being converted into other compounds. The hydrogen fluoride concentration is preferably 0.1 ppm by volume or more, and can be, for example, 0.2 ppm by volume or more or 0.3 ppm by volume or more. A hydrogen fluoride concentration of 0.1 ppm by volume or more can inhibit 1,1,1,2-tetrafluoroethane being stored from being decomposed. While not bound by any theory, the reason for this is considered because, while 1,1,1,2-tetrafluoroethane is decomposed into trifluoroethen, this decomposition reaction is an equilibrium reaction and the presence of hydrogen fluoride enables an equilibrium point to be biased toward 1,1,1,2-tetrafluoroethane. The hydrogen fluoride concentration can be 0.1 to 5.0 ppm by volume, preferably 0.1 to 3.0 ppm by volume, more preferably 0.1 to 1.0 ppm by volume, for example, 0.2 to 3.0 ppm by volume, 0.3 to 3.0 ppm by volume, 0.2 to 1.0 ppm by volume, or 0.3 to 1.0 ppm by volume.

The hydrogen chloride concentration in the composition is 5.0 ppm by volume or less, preferably 3.0 ppm by volume or less, more preferably 1.0 ppm by volume or less. A hydrogen chloride concentration of 5.0 ppm by volume or less can inhibit the fluorocarbon compound having a double bond from being converted into other compounds. The hydrogen chloride concentration is preferably 0.1 ppm by volume or more, and can be, for example, 0.2 ppm by volume or more or 0.3 ppm by volume or more. A hydrogen chloride concentration of 0.1 ppm by volume or more can allow a passive film to be formed in a container, resulting in an enhancement in storage stability of the composition. The hydrogen chloride concentration can be 0.1 to 5.0 ppm by volume, preferably 0.1 to 3.0 ppm by volume, more preferably 0.1 to 1.0 ppm by volume, for example, 0.2 to 3.0 ppm by volume, 0.3 to 3.0 ppm by volume, 0.2 to 1.0 ppm by volume, or 0.3 to 1.0 ppm by volume.

The concentration of the fluorocarbon compound having a double bond is preferably 50 ppm by volume or less, more preferably 20 ppm by volume or less, further preferably 10 ppm by volume or less, and can be, for example, 5.0 ppm by volume or less or 1.0 ppm by volume or less. The concentration of the fluorocarbon compound having a double bond is preferably 0.1 ppm by volume or more, and can be, for example, 0.2 ppm by volume or more or 0.2 ppm by volume or more. The concentration of the fluorocarbon compound having a double bond can be preferably 0.1 to 50 ppm by volume, preferably 0.1 to 20 ppm by volume, more preferably 0.1 to 10 ppm by volume, further preferably 0.1 to 5.0 ppm by volume.

The concentration of the 1,1,1,2-tetrafluoroethane is preferably 99.9990% by volume or more, more preferably 99.9999% by volume or more.

The present disclosure also provides a method for storing a composition including 1,1,1,2-tetrafluoroethane and a fluorocarbon compound having one or more double bonds, in which the oxygen concentration is controlled to be 5.0 ppm by volume or less.

The storage temperature can be preferably 60°C or less, more preferably 50°C or less, further preferably 40°C or less. A storage temperature of 60°C or less can inhibit the fluorocarbon compound having a double bond from being converted into other compounds. The storage temperature is preferably 0°C or more, and can be, for example, 10°C or more, 15°C or more or 20°C or more. The storage method of the present disclosure has no need for excess cooling because the fluorocarbon compound having a double bond can be sufficiently inhibited from being converted into other compounds even at 0°C or more. For example, the storage method may be performed at ordinary temperature. The storage temperature is preferably 0 to 60°C, preferably 0 to 50°C, and can be, for example, 10 to 50°C, or 20 to 50°C.

In a preferred embodiment, storage is performed in a dark place. For example, the container may be a light-blocking container, and the storage place may be a dark place.

### Examples

Hereinafter, a purification method in the present disclosure is more specifically described through the following Examples, but the present disclosure is not limited to these Examples.

### (Initial composition 1)

Prepared were compositions 1 to 9 each including 1,1,1,2-tetrafluoroethane (HFC-134a), (E/Z)1-chloro-1,2-difluoroethene (HFO-1122a), (E/Z)1-chloro-2-fluoroethene (HFO-1131), and 3,3,3-trifluoropropene (HFO-1243zf) at proportions shown in Table 1 below.

**[Table 1]**

| | Concentration (ppm by volume) | | |
|---|---|---|---|
| | 1122a | 1131 | 1243zf |
| Composition 1 | 15 | - | - |
| Composition 2 | 5 | - | - |
| Composition 3 | 1 | - | - |
| Composition 4 | - | 50 | - |
| Composition 5 | - | 10 | - |
| Composition 6 | - | 1 | - |
| Composition 7 | - | - | 20 |
| Composition 8 | - | - | 10 |
| Composition 9 | - | - | 1 |

### (Examples)

Compositions 1 to 9 described above were each prepared so as to have oxygen concentrations of 100 ppm by volume, 10 ppm by volume, 5 ppm by volume, and 1 ppm by volume, and stored at 20°C and 50°C. Each gas composition was analyzed by chromatography after 30 days, 90 days, and 180 days from the start of storage. The content of a component not included in the initial composition was calculated. The results at 20°C are shown in Table 2, and the results at 50°C are shown in Table 3. ND here represents a detection limit or less (0.1 ppm by volume or less).

**[Table 2]**

| | | 30 Days | 90 Days | 180 Days |
|---|---|---|---|---|
| | Oxygen concentration (ppm by volume) | Impurity concentration (ppm by volume) | | |
| Composition 1 | 100 | 0.8 | 1.3 | 2.9 |
| Composition 1 | 10 | ND | 0.8 | 0.9 |
| Composition 1 | 5 | ND | ND | ND |
| Composition 1 | 1 | ND | ND | ND |
| Composition 2 | 100 | 0.9 | 1.1 | 2.8 |
| Composition 2 | 10 | ND | 0.9 | 1.1 |
| Composition 2 | 5 | ND | ND | ND |
| Composition 2 | 1 | ND | ND | ND |
| Composition 3 | 100 | 0.6 | 0.8 | 1.1 |
| Composition 3 | 10 | ND | ND | 0.4 |
| Composition 3 | 5 | ND | ND | ND |
| Composition 3 | 1 | ND | ND | ND |
| Composition 4 | 100 | 1.1 | 1.5 | 2.1 |
| Composition 4 | 10 | ND | 0.9 | 1.1 |
| Composition 4 | 5 | ND | ND | ND |
| Composition 4 | 1 | ND | ND | ND |
| Composition 5 | 100 | 1.1 | 2.1 | 2.6 |
| Composition 5 | 10 | ND | 1.2 | 1.5 |
| Composition 5 | 5 | ND | ND | ND |
| Composition 5 | 1 | ND | ND | ND |
| Composition 6 | 100 | 0.2 | 1.3 | 2.4 |
| Composition 6 | 10 | ND | ND | 0.4 |
| Composition 6 | 5 | ND | ND | ND |
| Composition 6 | 1 | ND | ND | ND |
| Composition 7 | 100 | 3.6 | 5.6 | 8.9 |
| Composition 7 | 10 | ND | 1.5 | 2.6 |
| Composition 7 | 5 | ND | ND | ND |
| Composition 7 | 1 | ND | ND | ND |
| Composition 8 | 100 | 2.2 | 3.4 | 6.6 |
| Composition 8 | 10 | ND | 1.1 | 1.5 |
| Composition 8 | 5 | ND | ND | ND |
| Composition 8 | 1 | ND | ND | ND |
| Composition 9 | 100 | 1.3 | 2.4 | 3.1 |
| Composition 9 | 10 | ND | ND | 0.8 |
| Composition 9 | 5 | ND | ND | ND |
| Composition 9 | 1 | ND | ND | ND |

**[Table 3]**

| | | 30 Days | 90 Days | 180 Days |
|---|---|---|---|---|
| | Oxygen concentration (ppm by volume) | Impurity concentration (ppm by volume) | | |
| Composition 1 | 100 | 1.8 | 3.4 | 7.9 |
| Composition 1 | 10 | 0.4 | 0.9 | 1.6 |
| Composition 1 | 5 | ND | ND | ND |
| Composition 1 | 1 | ND | ND | ND |
| Composition 2 | 100 | 0.9 | 1.4 | 2.7 |
| Composition 2 | 10 | 0.4 | 0.8 | 1.4 |
| Composition 2 | 5 | ND | ND | ND |
| Composition 2 | 1 | ND | ND | ND |
| Composition 3 | 100 | 0.9 | 1.2 | 1.9 |
| Composition 3 | 10 | ND | 0.4 | 0.9 |
| Composition 3 | 5 | ND | ND | ND |
| Composition 3 | 1 | ND | ND | ND |
| Composition 4 | 100 | 2.2 | 4.2 | 5.5 |
| Composition 4 | 10 | 0.5 | 1.4 | 2.8 |
| Composition 4 | 5 | ND | ND | ND |
| Composition 4 | 1 | ND | ND | ND |
| Composition 5 | 100 | 1.9 | 3.4 | 5.5 |
| Composition 5 | 10 | 0.9 | 2.3 | 5.1 |
| Composition 5 | 5 | ND | ND | ND |
| Composition 5 | 1 | ND | ND | ND |
| Composition 6 | 100 | 2.3 | 3.5 | 6.3 |
| Composition 6 | 10 | ND | 0.4 | 0.5 |
| Composition 6 | 5 | ND | ND | ND |
| Composition 6 | 1 | ND | ND | ND |
| Composition 7 | 100 | 5.9 | 7.8 | 9.5 |
| Composition 7 | 10 | 1.2 | 3.3 | 6.5 |
| Composition 7 | 5 | ND | ND | ND |
| Composition 7 | 1 | ND | ND | ND |
| Composition 8 | 100 | 5.3 | 6.7 | 10.3 |
| Composition 8 | 10 | 0.8 | 2.2 | 5.4 |
| Composition 8 | 5 | ND | ND | ND |
| Composition 8 | 1 | ND | ND | ND |
| Composition 9 | 100 | 2.2 | 3.5 | 6.5 |
| Composition 9 | 10 | ND | 0.4 | 3.3 |
| Composition 9 | 5 | ND | ND | ND |
| Composition 9 | 1 | ND | ND | ND |

### (Example 2)

Compositions 1 to 9 described above were each prepared so as to have hydrogen fluoride concentrations of 100 ppm by volume, 10 ppm by volume, 5 ppm by volume, and 1 ppm by volume, and stored at 20°C and 50°C. Each gas composition was analyzed by chromatography after 30 days, 90 days, and 180 days from the start of storage. The content of a component not included in the initial composition was calculated. The results at 20°C are shown in Table 4 and the results at 50°C are shown in Table 5. ND here represents a detection limit or less (0.1 ppm by volume or less).

**[Table 4]**

| 20°C | | 30 Days | 90 Days | 180 Days |
|---|---|---|---|---|
| | HF concentration (ppm by volume) | Impurity concentration (ppm by volume) | | |
| Composition 1 | 100 | 5.3 | 11 | 21 |
| Composition 1 | 10 | ND | 9 | 18 |
| Composition 1 | 5 | ND | ND | ND |
| Composition 1 | 1 | ND | ND | ND |
| Composition 2 | 100 | 2.2 | 4.9 | 11 |
| Composition 2 | 10 | ND | 1.0 | 2.4 |
| Composition 2 | 5 | ND | ND | ND |
| Composition 2 | 1 | ND | ND | ND |
| Composition 3 | 100 | 0.4 | 0.8 | 1.2 |
| Composition 3 | 10 | ND | ND | 0.9 |
| Composition 3 | 5 | ND | ND | ND |
| Composition 3 | 1 | ND | ND | ND |
| Composition 4 | 100 | 1.1 | 1.7 | 1.9 |
| Composition 4 | 10 | ND | 0.9 | 1.1 |
| Composition 4 | 5 | ND | ND | ND |
| Composition 4 | 1 | ND | ND | ND |
| Composition 5 | 100 | 0.9 | 1.1 | 1.4 |
| Composition 5 | 10 | ND | 0.6 | 1.1 |
| Composition 5 | 5 | ND | ND | ND |
| Composition 5 | 1 | ND | ND | ND |
| Composition 6 | 100 | 0.4 | 0.6 | 0.8 |
| Composition 6 | 10 | ND | ND | 0.7 |
| Composition 6 | 5 | ND | ND | ND |
| Composition 6 | 1 | ND | ND | ND |
| Composition 7 | 100 | 4.3 | 9.6 | 13.2 |
| Composition 7 | 10 | ND | 4.4 | 10.4 |
| Composition 7 | 5 | ND | ND | ND |
| Composition 7 | 1 | ND | ND | ND |
| Composition 8 | 100 | 1.1 | 6.6 | 10.1 |
| Composition 8 | 10 | ND | 3.2 | 7.5 |
| Composition 8 | 5 | ND | ND | ND |
| Composition 8 | 1 | ND | ND | ND |
| Composition 9 | 100 | 1.3 | 2.4 | 2.8 |
| Composition 9 | 10 | ND | ND | 0.9 |
| Composition 9 | 5 | ND | ND | ND |
| Composition 9 | 1 | ND | ND | ND |

**[Table 5]**

| 50°C | | 30 Days | 90 Days | 180 Days |
|---|---|---|---|---|
| | HF concentration (ppm by volume) | Impurity concentration (ppm by volume) | | |
| Composition 1 | 100 | 8.3 | 22 | 46 |
| Composition 1 | 10 | 2.2 | 3.3 | 6.6 |
| Composition 1 | 5 | ND | ND | ND |
| Composition 1 | 1 | ND | ND | ND |
| Composition 2 | 100 | 18 | 21 | 33 |
| Composition 2 | 10 | 17 | 21 | 29 |
| Composition 2 | 5 | ND | ND | ND |
| Composition 2 | 1 | ND | ND | ND |
| Composition 3 | 100 | 1.1 | 1.7 | 3.1 |
| Composition 3 | 10 | ND | 0.9 | 1.4 |
| Composition 3 | 5 | ND | ND | ND |
| Composition 3 | 1 | ND | ND | ND |
| Composition 4 | 100 | 1.3 | 2.2 | 3.4 |
| Composition 4 | 10 | 0.8 | 1.1 | 1.4 |
| Composition 4 | 5 | ND | ND | ND |
| Composition 4 | 1 | ND | ND | ND |
| Composition 5 | 100 | 1.5 | 2.2 | 2.8 |
| Composition 5 | 10 | 0.6 | 1.5 | 1.6 |
| Composition 5 | 5 | ND | ND | ND |
| Composition 5 | 1 | ND | ND | ND |
| Composition 6 | 100 | 0.5 | 1.1 | 1.9 |
| Composition 6 | 10 | ND | 0.6 | 1.4 |
| Composition 6 | 5 | ND | ND | ND |
| Composition 6 | 1 | ND | ND | ND |
| Composition 7 | 100 | 5.5 | 18.7 | 22.1 |
| Composition 7 | 10 | 3.3 | 5.7 | 12.2 |
| Composition 7 | 5 | ND | ND | ND |
| Composition 7 | 1 | ND | ND | ND |
| Composition 8 | 100 | 4.9 | 8.1 | 11.3 |
| Composition 8 | 10 | 3.4 | 5.5 | 9.3 |
| Composition 8 | 5 | ND | ND | ND |
| Composition 8 | 1 | ND | ND | ND |
| Composition 9 | 100 | 2.2 | 4.9 | 7.5 |
| Composition 9 | 10 | ND | 1.6 | 3.8 |
| Composition 9 | 5 | ND | ND | ND |
| Composition 9 | 1 | ND | ND | ND |

### (Example 3)

Compositions 1 to 9 described above were each prepared so as to have hydrogen chloride concentrations of 100 ppm by volume, 10 ppm by volume, 5 ppm by volume, and 1 ppm by volume, and stored at 20°C and 50°C. Each gas composition was analyzed by chromatography after 30 days, 90 days, and 180 days from the start of storage. The content of a component not included in the initial composition was calculated. The results at 20°C are shown in Table 6 and the results at 50°C are shown in Table 7. ND here represents a detection limit or less (0.1 ppm by volume or less).

**[Table 6]**

| 20°C | | 30 Days | 90 Days | 180 Days |
|---|---|---|---|---|
| | HCl concentration (ppm by volume) | Impurity concentration (ppm by volume) | | |
| Composition 1 | 100 | 1.3 | 2.7 | 5.3 |
| Composition 1 | 10 | | 1.5 | 4.8 |
| Composition 1 | 5 | ND | ND | ND |
| Composition 1 | 1 | ND | ND | ND |
| Composition 2 | 100 | 0.5 | 2.4 | 4.3 |
| Composition 2 | 10 | | 1.3 | 2.7 |
| Composition 2 | 5 | ND | ND | ND |
| Composition 2 | 1 | ND | ND | ND |
| Composition 3 | 100 | 0.7 | 0.6 | 1.1 |
| Composition 3 | 10 | | | 0.6 |
| Composition 3 | 5 | ND | ND | ND |
| Composition 3 | 1 | ND | ND | ND |
| Composition 4 | 100 | 0.9 | 1.4 | 2.2 |
| Composition 4 | 10 | | 0.9 | 1.3 |
| Composition 4 | 5 | ND | ND | ND |
| Composition 4 | 1 | ND | ND | ND |
| Composition 5 | 100 | 1.2 | 2.8 | 4.2 |
| Composition 5 | 10 | | 0.7 | 0.9 |
| Composition 5 | 5 | ND | ND | ND |
| Composition 5 | 1 | ND | ND | ND |
| Composition 6 | 100 | 0.6 | 1.5 | 2.5 |
| Composition 6 | 10 | | : | 0.8 |
| Composition 6 | 5 | ND | ND | ND |
| Composition 6 | 1 | ND | ND | ND |
| Composition 7 | 100 | 4.4 | 6.2 | 13.3 |
| Composition 7 | 10 | | 5.7 | 10.4 |
| Composition 7 | 5 | ND | ND | ND |
| Composition 7 | 1 | ND | ND | ND |
| Composition 8 | 100 | 1.4 | 8.6 | 11.4 |
| Composition 8 | 10 | | 3.2 | 8.9 |
| Composition 8 | 5 | ND | ND | ND |
| Composition 8 | 1 | ND | ND | ND |
| Composition 9 | 100 | 1.1 | 2.2 | 3.2 |
| Composition 9 | 10 | | | 1.6 |
| Composition 9 | 5 | ND | ND | ND |
| Composition 9 | 1 | ND | ND | ND |

**[Table 7]**

| 50°C | | 30 Days | 90 Days | 180 Days |
|---|---|---|---|---|
| | HCl concentration (ppm by volume) | Impurity concentration (ppm by volume) | | |
| Composition 1 | 100 | 2.2 | 5.5 | 7.7 |
| Composition 1 | 10 | 1.4 | 3.5 | 4.9 |
| Composition 1 | 5 | ND | ND | ND |
| Composition 1 | 1 | ND | ND | ND |
| Composition 2 | 100 | 1.5 | 3.2 | 5.8 |
| Composition 2 | 10 | 0.9 | 2.1 | 3.5 |
| Composition 2 | 5 | ND | ND | ND |
| Composition 2 | 1 | ND | ND | ND |
| Composition 3 | 100 | 1.5 | 2.1 | 3.2 |
| Composition 3 | 10 | ND | 0.7 | 1.7 |
| Composition 3 | 5 | ND | ND | ND |
| Composition 3 | 1 | ND | ND | ND |
| Composition 4 | 100 | 1.7 | 2.4 | 3.5 |
| Composition 4 | 10 | 0.7 | 1.4 | 2.8 |
| Composition 4 | 5 | ND | ND | ND |
| Composition 4 | 1 | ND | ND | ND |
| Composition 5 | 100 | 2.3 | 4.8 | 8.3 |
| Composition 5 | 10 | 0.6 | 1.3 | 1.7 |
| Composition 5 | 5 | ND | ND | ND |
| Composition 5 | 1 | ND | ND | ND |
| Composition 6 | 100 | 1.2 | 2.4 | 3.7 |
| Composition 6 | 10 | ND | 0.7 | 1.1 |
| Composition 6 | 5 | ND | ND | ND |
| Composition 6 | 1 | ND | ND | ND |
| Composition 7 | 100 | 6.5 | 18 | 24.4 |
| Composition 7 | 10 | 4.3 | 10.2 | 13.4 |
| Composition 7 | 5 | ND | ND | ND |
| Composition 7 | 1 | ND | ND | ND |
| Composition 8 | 100 | 4.8 | 9.9 | 16.5 |
| Composition 8 | 10 | 2.4 | 5.5 | 10.9 |
| Composition 8 | 5 | ND | ND | ND |
| Composition 8 | 1 | ND | ND | ND |
| Composition 9 | 100 | 3.3 | 5.4 | 8.5 |
| Composition 9 | 10 | ND | 2.8 | 4.5 |
| Composition 9 | 5 | ND | ND | ND |
| Composition 9 | 1 | ND | ND | ND |

Example 4

(E/Z)1-chloro-1,2-difluoroethene (HFO-1122a), (E/Z)1-chloro-2-fluoroethene (HFO-1131), and 3,3,3-trifluoropropene (HFO-1243zf) were each prepared so as to have oxygen concentrations of 0.1 ppm by volume and 0.01 ppm by volume, and stored at 20°C and 50°C. Each gas composition was analyzed by chromatography after 30 days, 90 days, and 180 days from the start of storage. The content of a component not included in the initial composition was calculated. The results at 20°C are shown in Table 9 and the results at 50°C are shown in Table 10.

**[Table 8]**

| | Component | Oxygen |
|---|---|---|
| Composition 10 | 1122a | 0.1 |
| Composition 11 | 1122a | 0.01 |
| Composition 12 | 1131 | 0.1 |
| Composition 13 | 1131 | 0.01 |
| Composition 14 | 1243zf | 0.1 |
| Composition 15 | 1243zf | 0.01 |

**[Table 9]**

| 20°C | 30 Days | 90 Days | 180 Days |
|---|---|---|---|
| | Concentration of decomposed product (ppm by volume) | | |
| Composition 10 | ND | ND | ND |
| Composition 11 | ND | 0.3 | 0.4 |
| Composition 12 | ND | ND | ND |
| Composition 13 | ND | 0.4 | 0.6 |
| Composition 14 | ND | ND | ND |
| Composition 15 | ND | 0.6 | 0.7 |

**[Table 10]**

| 50°C | 30 Days | 90 Days | 180 Days |
|---|---|---|---|
| | Concentration of decomposed product (ppm by volume) | | |
| Composition 10 | ND | ND | ND |
| Composition 11 | ND | 0.4 | 0.5 |
| Composition 12 | ND | ND | ND |
| Composition 13 | ND | 0.4 | 0.7 |
| Composition 14 | ND | ND | ND |
| Composition 15 | ND | 0.7 | 0.8 |

### (Examples 5 and 6)

1,1,1,2-Tetrafluoroethane (HFC-134a) was purified to obtain high-purity HFC-134a where no component other than HFC-134a was detected by gas chromatography, and hydrogen fluoride was added thereto so as to have a concentration of 0.1 ppm by volume. The resultant portions were stored at 20°C and 50°C. Each gas composition was analyzed by chromatography after 30 days, 90 days, and 180 days from the start of storage. The results at 20°C are shown in Table 11 and the results at 50°C are shown in Table 12.

### (Comparative Examples 1 and 2)

No hydrogen fluoride was added, and high-purity HFC-134a was stored at 20°C and 50°C. Each gas composition was analyzed by chromatography after 30 days, 90 days, and 180 days from the start of storage. The results at 20°C are shown in Table 4 and the results at 50°C are shown in Table 5.

**[Table 11]**

| 20°C | | 30 Days | 90 Days | 180 Days |
|---|---|---|---|---|
| | HF concentration (ppm by volume) | Trifluoroethene (ppm by volume) | | |
| Example 5 | 0.1 | N.D. | N.D. | N.D. |
| Comparative Example 1 | N.D. | 0.05 | 0.07 | 0.08 |

**[Table 12]**

| 50°C | | 30 Days | 90 Days | 180 Days |
|---|---|---|---|---|
| | HF concentration (ppm by volume) | Trifluoroethene (ppm by volume) | | |
| Example 6 | 0.1 | N.D. | N.D. | N.D. |
| Comparative Example 2 | N.D. | 0.08 | 0.10 | 0.12 |

### Industrial Applicability

The composition including 1,1,1,2-tetrafluoroethane of the present disclosure is suitable for long storage.

## Claims

1. A composition comprising 1,1,1,2-tetrafluoroethane and a fluorocarbon compound having one or more double bonds, wherein an oxygen concentration, a hydrogen fluoride concentration, or a hydrogen chloride concentration is 5.0 ppm by volume or less.

2. The composition according to claim 1, wherein the oxygen concentration is 5.0 ppm by volume or less.

3. The composition according to claim 1, wherein the hydrogen fluoride concentration is 5.0 ppm by volume or less.

4. The composition according to claim 1, wherein the hydrogen chloride concentration is 5.0 ppm by volume or less.

5. The composition according to any one of claims 1 to 4, wherein the fluorocarbon compound having one or more double bonds has one double bond.

6. The composition according to any one of claims 1 to 5, wherein the fluorocarbon compound having one or more double bonds has 2 or 3 carbon atoms.

7. The composition according to any one of claims 1 to 6, wherein the fluorocarbon compound having one or more double bonds comprises at least one compound selected from the group consisting of (E/Z)1-chloro-1,2-difluoroethene, (E/Z)1-chloro-2-fluoroethene, and 3,3,3-trifluoropropene.

8. The composition according to any one of claims 1 to 7, wherein the oxygen concentration, the hydrogen fluoride concentration, or the hydrogen chloride concentration is 0.1 ppm by volume or more and 5.0 ppm by volume or less.

9. The composition according to any one of claims 1 to 8, wherein the oxygen concentration, the hydrogen fluoride concentration, or the hydrogen chloride concentration is 0.1 ppm by volume or more and 1.0 ppm by volume or less.

10. The composition according to any one of claims 1 to 9, wherein a concentration of the fluorocarbon compound having one or more double bonds is 0.1 ppm by volume or more and 50 ppm by volume or less.

11. The composition according to any one of claims 1 to 10, wherein a concentration of the fluorocarbon compound having one or more double bonds is 0.1 ppm by volume or more and 2.0 ppm by volume or less.

12. The composition according to any one of claims 1 to 11, wherein a concentration of the 1,1,1,2-tetrafluoroethane is 99.9999% by volume or more.

13. A method for storing a composition comprising 1,1,1,2-tetrafluoroethane and a fluorocarbon compound having one or more double bonds, wherein an oxygen concentration, a hydrogen fluoride concentration, or a hydrogen chloride concentration is set to 5.0 ppm by volume or less.

14. The storage method according to claim 13, wherein the oxygen concentration, the hydrogen fluoride concentration, or the hydrogen chloride concentration is 0.1 ppm by volume or more and 5.0 ppm by volume or less.

15. The storage method according to claim 13 or 14, wherein a storage temperature is 50°C or less.

16. The storage method according to claim 13 or 14, wherein a storage temperature is 0°C or more and 50°C or less.
